# EUROPEAN PATENT APPLICATION

(11) **EP 0 689 847 A1**
(43) Date of publication of application: **03.01.1996**
(21) Application number: 95304415.3
(22) Date of filing: 23.06.1995
(51) Int. Cl.: A61M 5/315

(54) **Syringe assembly**

(30) Priority: 01.07.1994 US 269719
(71) Applicant: Becton Dickinson and Company, Franklin Lakes, New Jersey 07417-1880 (US)
(72) Inventor: Watts, Lennox O., Bronx, New York 10466 (US)
(74) Representative: Ruffles, Graham Keith

(57) **Abstract**

A syringe assembly (10) includes a barrel (12) and a plunger (40) slidably placed in the barrel (12). The plunger (40) includes a shaft with a distal piston (46). The piston (46) has a distal sealing portion (48) with a distal surface (54) including a central section (56) and an outside section (58) and a guide portion (50). The sealing portion (48) has an outside diameter greater than the inside diameter of the barrel before the piston is placed in the barrel. The sealing portion (48) has a groove (62) in the distal surface (54) where the central section (56) flexibly joins the outside section (58). When the piston (46) is placed in the barrel (12), the outside section ((58) contacts the barrel wall distally, deflecting the outside section (58) with respect to the central section (56), forming a fluid tight seal with the barrel wall for facilitating drawing and expelling fluid with movement of the piston (46). The guide portion (50) contacts the barrel wall to coaxially maintain the plunger (40) in the barrel (12).

The area of reduced thickness (60) in the plunger sealing portion is formed as a living hinge by the part being distally flexed at the area of reduced thickness as the mold is opened. The plunger molding tool has a primary and a secondary opening mode. In the method of forming the plunger, the mold first opens in the secondary mode forming the living hinge, and then opens in the primary mode providing access to the part.

## Description

### Field of the Invention

The present invention relates to a syringe assembly and more particularly to a syringe assembly having a plunger integrally formed from a single material and a method for forming the plunger.

### Background of the Invention

Generally speaking, a hypodermic syringe consists of a cylindrical barrel, most commonly made of thermoplastic materials or glass, having a distal end connected to a sharp needle cannula or adapted to be connected to a hypodermic needle assembly or other medical access device and a proximal open end adapted to receive a resilient stopper and a plunger rod assembly. One of the purposes of the stopper is to provide a substantially air or fluid tight seal between the barrel and itself so that movement of the stopper up and down the barrel will cause a liquid medication, blood or other fluid to be drawn into or forced out of the syringe through the distal end. The stopper is moved along the syringe barrel by applying axial force to the rigid plunger rod which is connected to the stopper and is sufficiently long as to be accessible outside of the barrel. The stopper should be sufficiently flexible so that it will seal the inside diameter of the barrel without requiring excessive force to move it up and down the barrel.

In order to assure a fluid-tight seal between the rigid syringe barrel and the resilient stopper, syringe plunger stoppers are manufactured with a larger outside diameter than the inside diameter of the syringe barrels where they will be used. The syringe/stopper combination must be designed so that sufficient compression is produced in the stopper that an adequate seal exists, but there must not be too much compression, because the stopper must move easily in the barrel. The most commonly used materials to produce resilient stoppers for syringes are thermoset elastomers. These resilient stoppers are generally formed from thermoset elastomers by compression molding which forms the stopper and cures the elastomer. Stoppers formed from thermoset elastomers often constitute a significant portion of the cost of a single use syringe, because they require separate forming equipment from the other components of a single use syringe and additionally, multiple steps and additional equipment for manufacture and assembly onto the syringe plunger. Lubricants such as polydimethylsiloxane are commonly used in syringe barrels to facilitate the movement of the resilient stopper against the rigid thermoplastic wall of the syringe barrel.

Attempts have been made to reduce the size of the resilient stopper and to produce syringes which do not have a separate plunger which must be separately manufactured, then subsequently mounted onto the plunger rod for assembly into the syringe barrel. U.S. Patent No. 4,500,310 to Christinger teaches an improved plunger rod design which allows the use of a smaller resilient thermoplastic stopper. Christinger overcomes many of the cost and possible functional problems related to thermoset rubber stoppers but still requires an additional elastomeric stopper element to be connected to the plunger rod.

An alternative to the use of an elastomeric stopper in a syringe assembly is disclosed by Dragosits in U.S. Patent No. 5,061,252. Dragosits discloses a syringe assembly having an extruded syringe barrel having a rigid outer layer and a coextruded resilient inner layer coupled with an rigid plunger. The rigid plunger forms a fluid tight seal by deflecting the resilient inner layer of the barrel. The Dragosits design requires that a barrel end piece be formed separately. Also, the extruded barrel must be precisely cut to length from the extruded tube and the end piece accurately placed in the barrel.

Syringe assemblies having a rigid plunger and a deflectable barrel are commercially available in Europe. These syringe assemblies form a fluid tight seal by deflection of the barrel wall by the rigid plunger. If a syringe of the European design is held between the thumb and the forefinger and the plunger is moved past the area where the fingers grasp the barrel, a ripple can be felt in the barrel wall as the plunger passes. The barrel wall must have a thin cross-section so that the barrel can deflect to form the seal, thus syringe barrels of this design may develop longitudinal stress cracks, and barrels may be easily flexed .

In the U.S., the Air-Tite company has offered syringes with a rigid barrel and a plunger having a thin tapered flange distal tip which is expected to form a fluid tight seal with the barrel wall. However, these syringes have been the subject of recall campaigns because of leakage between the barrel wall and piston which likely resulted from the tapered thin flange of the plunger being permanently distorted because of the compression between the flange and the barrel wall.

Although these alternatives to the generally used syringe assemblies having thermoset elastomeric plunger stoppers have been disclosed as described above, there is still a need for a syringe assembly which would be simply formed and would allow most existing designs and tooling for barrels and assembly equipment to remain usable. Such a design would eliminate the need for the cost and complexity associated with the currently used thermoplastic or thermoset elastomeric plunger stopper.

### Summary of the Invention

A syringe assembly of the present invention includes a barrel with a cylindrical body portion having a longitudinal axis. The body portion forms an inside wall spaced from the axis which defines a chamber having an inside diameter for retaining fluid. The body portion has an open proximal end and a distal end with a passageway therethrough that is in fluid communication with the chamber. The syringe assembly also includes a plunger which has an elongate shaft with a longitudinal axis. The plunger has a proximal end and a distal end which includes a piston. The piston is slidably positioned in the barrel and includes a sealing portion and a guide portion. The sealing portion has a thickness and is located distally to the guide portion substantially transverse to the longitudinal axis of the plunger. The sealing portion has a distal surface and additionally includes a central section and an outside section. The sealing portion has an outside diameter greater than the inside diameter of the chamber before the piston is placed in the barrel. The sealing portion has an area of reduced thickness in the form of a groove in the distal surface which serves to flexibly join the central section and the outside section so that at least a circumferentially continuous portion of the outside section contacts the barrel wall and flexibly distally deflects the outside section relative to the central section at the area of reduced thickness allowing the piston to be fit within the barrel. The piston forms a fluid tight with the barrel wall and facilitates drawing fluid into and expelling fluid from the chamber with movement of the piston. The guide portion of the piston includes a plurality of projections which contact the inside wall of the chamber and maintain the plunger axis substantially coaxial with the barrel axis, the proximal end of the plunger extends outwardly from the proximal end of the barrel to facilitate moving the piston with respect to the barrel.

A plunger of the present invention is useful for placement within a syringe barrel having an inside wall and a diameter for forming a chamber within the barrel for facilitating drawing fluid into and expelling fluid from the chamber. The plunger includes an elongate shaft portion having a longitudinal axis, a proximal end and a distal end comprising an integrally formed piston. The piston includes a distal sealing portion and a guide portion and is slidably positioned in the barrel. The sealing portion has a thickness and is located distally to the guide portion. The sealing portion has a distal surface and includes a central section and an outside section. The sealing portion has an outside diameter greater than the inside diameter of the chamber and has an area of reduced thickness in the form of a groove in the distal surface. The area of reduced thickness flexibly joins the central section and the outside section so that when said piston is placed in the barrel at least a circumferentially continuous portion of the outside section contacts the inside wall and flexibly distally deflects the outside section relative to said central section at said area of reduced thickness. The deflection allows the piston to fit within the barrel and form a fluid tight seal with the inside wall of the barrel which facilitates drawing fluid into and expelling fluid from the chamber with movement of the piston. The guide portion of the piston includes a plurality of projections for contacting the inside wall of the barrel for maintaining said plunger axis substantially coaxial to the barrel axis. The proximal end of the plunger extends proximally outwardly from the barrel to facilitate moving the piston with respect to the barrel.

A method for making the plunger of the present invention includes providing an injection molding tool having a primary mode and a secondary mode of opening. These modes of opening provide the plunger with a primary parting line along the longitudinal axis corresponding to the elongate shaft and a secondary parting line on the sealing portion of the piston transverse to the longitudinal axis located proximally to the circumferentially continuous portion of the outside section which contacts the barrel wall. The method further includes providing an injection molding press and mounting the molding tool in the press. The press is charged with a thermoplastic resin and operated to melt the resin and fill the molding tool with resin. The resin is allowed to cool and the molding tool is opened in the secondary mode of opening so that the distal surface of the plunger is exposed. The secondary opening causes a distal flexion of the outside section of the sealing section of the piston with respect to said central section at the area of reduced thickness thereby forming a living hinge at the area of reduced thickness. The mold tool is then opened in the primary mode providing the plunger.

### Brief Description of the Drawings

Fig. 1 is an exploded perspective view of a syringe assembly of the present invention;
Fig. 2 is a plan view of the distal end of a syringe plunger of the present invention;
Fig. 3 shows an enlarged partial cross-section of the syringe assembly of Fig. 1 before the plunger is assembled into the barrel;
Fig. 4 shows an enlarged partial cross-section of the syringe assembly of Fig. 1 as the plunger is assembled into the barrel;
Fig. 5 shows an enlarged partial cross-section of the syringe assembly of Fig. 1 with the plunger assembled into the barrel;
Fig. 6 is a schematic cross-sectional view of the distal sealing portion of the plunger showing a portion of the mold tool used to form the plunger before the tool is opened;
Fig. 7 is a schematic cross-sectional view of the distal sealing portion of plunger showing a portion of the mold tool used to form the plunger at the start of the opening sequence;
Fig. 8 is a schematic cross-sectional view of the distal sealing portion of the plunger showing a portion of the mold tool used to form the plunger in a step of the opening sequence;
Fig. 9 is a side elevation view of the plunger of the present invention; and
Fig. 10 shows a cross-section of the plunger of the syringe assembly of Fig. 1 at the guide portion along the line 10,10 of Fig. 9.

### Detailed Description of the Invention

While this invention is satisfied by embodiments in may different forms, there is shown in the drawings and will herein be described in detail preferred embodiments of the invention with the understanding that the present disclosure is to be considered exemplary of the principles of the invention and is not intended to limit the invention to the embodiments illustrated. The scope of the invention will be measured by the appended claims and their equivalents.

For the purposes of the description of the present invention, the term "distal end" is meant to refer to the end of the syringe assembly closest to the needle cannula or to the portion of the syringe assembly where a needle cannula or other medical access device may be attached. The term "proximal end" refers to the end of the syringe assembly furthest from that portion of the syringe assembly having the needle cannula or other medical access device.

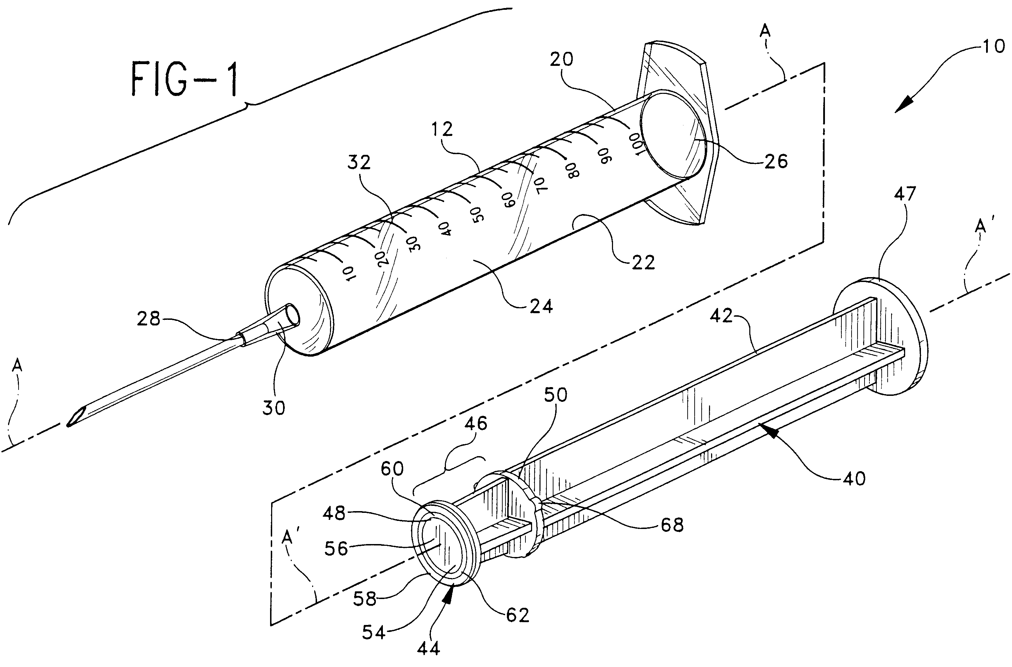

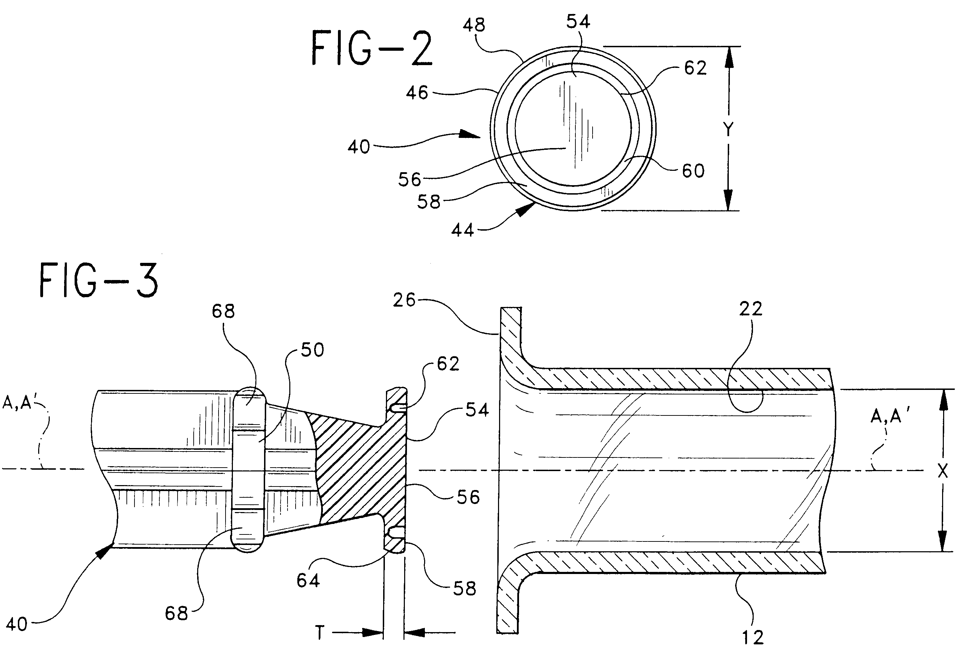

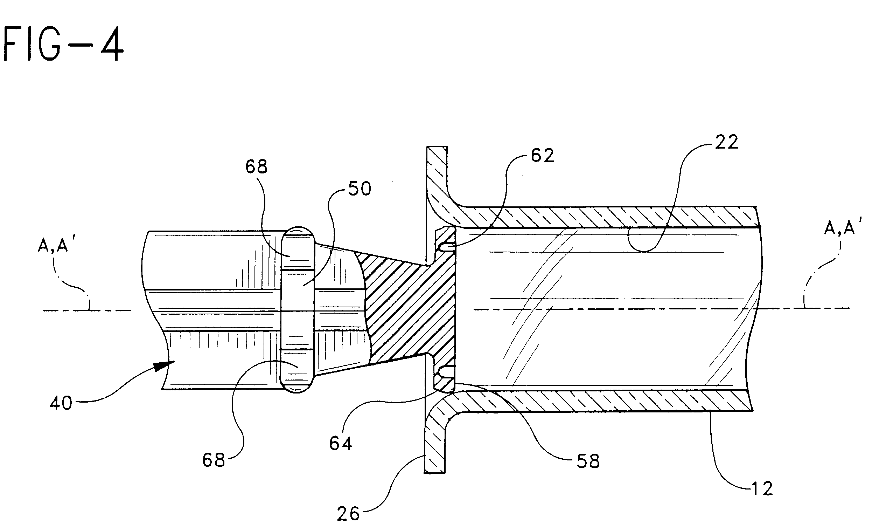

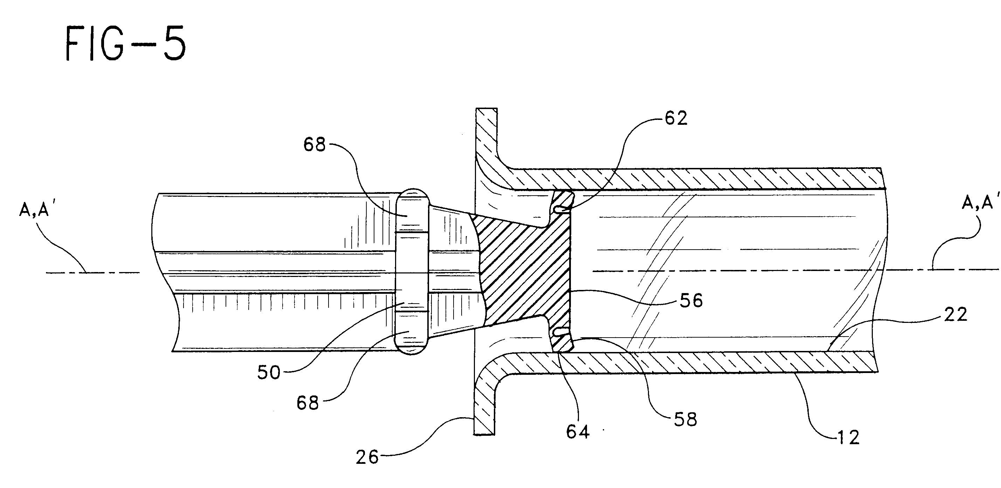

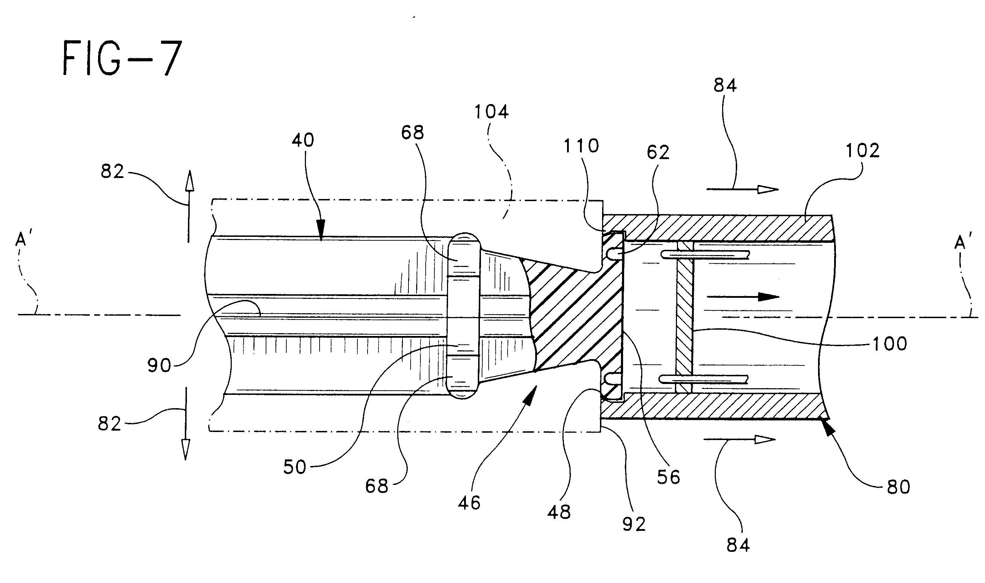

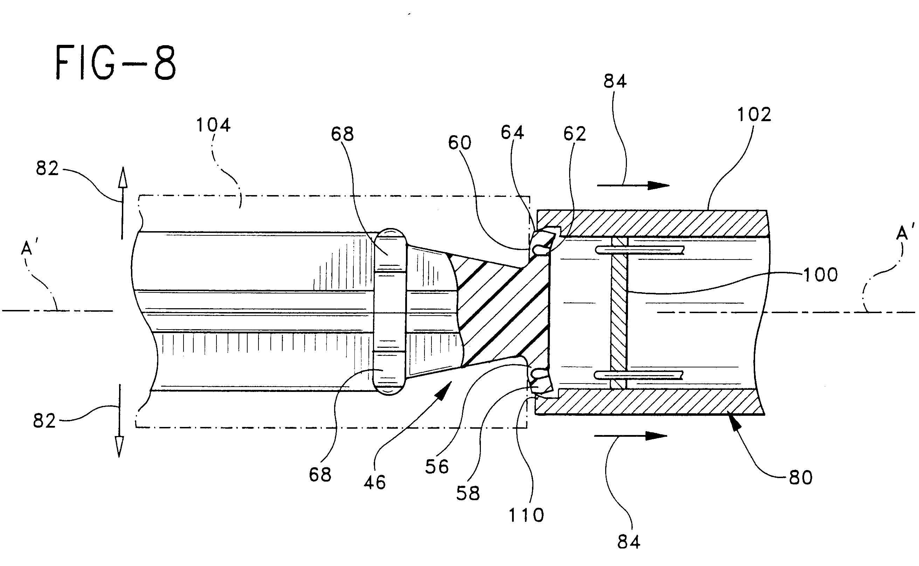

Referring to the Figs. 1-10, a syringe assembly 10 of the present invention includes barrel 12 which has a cylindrical body portion 20 having a longitudinal axis A. Body portion 20 has an inside wall 22 spaced from Axis A defining a chamber 24 with an inside diameter X for retaining fluid. Body portion 20 further includes an open proximal end 26 for retaining fluid and a distal end 28 having a passageway 30 therethrough in fluid communication with chamber 24. Assembly 10 further includes a plunger 40 with an elongate shaft 42 and having a longitudinal axis A'. Plunger 40 has a distal end 44 which includes a piston 46 and a proximal end 47. Piston 46 is preferably slidably positioned in barrel 12. Piston 46 has a distal sealing portion 48 and a guide portion 50. Sealing portion 48 has a thickness T and is located distally to guide portion 50. Sealing portion 46 has a distal surface 54 and includes a central section 56 and an outside section 58. Sealing portion 48 has an outside diameter Y which is greater than inside diameter X of chamber 24 before piston 46 is placed in barrel 12. Sealing portion 48 has an area of reduced thickness 60 in the form of a groove 62 in distal surface 54. The area of reduced thickness 60 flexibly joins central section 56 to outside section 58. When piston 46 is placed in barrel 12, at least a circumferentially continuous portion 64 of outside section 58 contacts barrel wall 22 flexibly distally deflecting outside section 58 relative to central section 56 allowing piston 46 to fit within barrel 12 thereby forming a fluid tight seal 66 with barrel wall 22. By forming a fluid tight seal with the barrel wall, piston 46 facilitates drawing fluid into and expelling fluid from chamber 24 with movement of the piston. Guide portion 50 includes a plurality of projections 68 to contact barrel wall 22 and maintain plunger axis A' substantially coaxial with barrel axis A. Preferably, plunger proximal end 47 extends outwardly from the barrel to facilitate moving piston 46 with respect to the barrel.

Preferably, distal end 28 of the barrel has provisions for mounting a cannula 70 or other medical access device in fluid communication with passageway 30. The provision for mounting a cannula may be a male luer fitting or other suitable fittings such as threads, snap-fit and the like. The medical access device such as a cannula also may be fixedly attached to distal end 28 of the barrel by adhesive bonding, mechanical press-fit or any other bonding technique suitable for medical devices. In the case where adhesive bonding of a cannula is selected, heat cured and photo cured epoxy resins are preferred. Preferably, barrel 12 is substantially transparent, and includes a scale 32 providing measuring indicia for determining the amount of fluid in the chamber.

The area of reduced thickness 60 which allows outside section 58 to deflect distally with respect to central section 56 is preferably in the form of a living hinge. In the art of injection molding, when an article formed from certain polymers has an area of reduced thickness relative to other portions of the article, a living hinge can be formed by flexing the article at area of the reduced thickness after it has been freshly removed from the molding tool. The immediate flexion of the article at the area of reduced thickness serves to orient the molecules within the flex area and allow the article subsequently be repeatedly flexed at the area of reduced thickness with no degradation of properties. If the article is not flexed when it is freshly removed from the mold, the ability to form the hinge at the area of reduced thickness is lost because the molecules will not be oriented as the article is flexed. Hence the name "living hinge". Polyethylene, polypropylene and copolymers of polypropylene are particularly well suited for the formation of a living hinge. Preferably the area of reduced thickness 60 is formed as a living hinge. The formation of the living hinge at area of reduced thickness 60 is accomplished by the design of the plunger, the design of the molding tool and the procedure for opening the molding tool. Preferably, the plunger of the present invention is formed by injection molding from polypropylene and copolymers of polypropylene.

In manufacture of syringe assemblies with resilient piston plungers, it is important that the material used for the piston not take a compression set during the period after assembly and before use. Compression set for a resilient material may be defined as subjecting the resilient material to a load for some period of time which causes it to be deformed from its unloaded state. The load is then removed and a determination is made of how much of its original form is regained. A material with no compression set property will rapidly return to its original form when the load is removed, while a material which takes a compression set will tend to retain the deformation acquired when loaded, even though the load is removed.

In the design of the plunger sealing surface of the present invention, it was recognized that a stress concentration would occur in the area of reduced thickness 60 when the plunger was inserted into the barrel and that it was necessary for a compression force be maintained between the plunger and the barrel. The compression force provides the stress concentration in the area of reduced thickness. It was further recognized that in order for the plunger sealing surface to provide an acceptable fluid tight seal for drawing and expelling fluid from the chamber, the compression force applied to the area of reduced thickness forming the stress concentration must not be greater than the yield point of the polymer. If the polymer is stressed beyond its yield point it will be permanently deformed at the area of reduced thickness and the plunger will lose its ability to maintain a fluid tight seal. A table of dimensions for common commercial sizes of single use syringe assemblies of the present invention is given below.

**Table I**

| Syringe Size(ml) | Barrel I.D. (inches) | Central Section O.D. (inches) | Outside Section O.D.(inches) | Guide Portion O.D. (inches) |
|---|---|---|---|---|
| 0.3 | 0.120 | 0.060 | 0.125 | 0.123 |
| 0.5 | 0.140 | 0.080 | 0.145 | 0.143 |
| 1 | 0.185 | 0.1246 | 0.190 | 0.188 |
| 2 | 0.338 | 0.278 | 0.343 | 0.341 |
| 3 | 0.338 | 0.278 | 0.343 | 0.341 |
| 5 | 0.472 | 0.414 | 0.479 | 0.477 |
| 10 | 0.568 | 0.508 | 0.573 | 0.571 |
| 20 | 0.750 | 0.690 | 0.755 | 0.753 |
| 30 | 0.850 | 0.791 | 0.856 | 0.854 |
| 60 | 1.047 | 0.989 | 1.054 | 1.052 |

When a comparison is made between the inside diameter (I.D.) of the barrel and the outside diameter (O.D.) of the outside section of the plunger for the various syringe sizes, it is apparent that the plunger outside section O.D. is greater than the barrel I.D. This overlap is referred to as an interference fit. This interference fit is what provides the compression force to deflect the outside section of the plunger with respect to the central section of the plunger thereby forming fluid tight seal 66 between the outside section of the piston and the barrel wall. Preferably the interference fit, i.e., the overlap between the barrel I.D. and the Outside section O.D. will be between about 0.001 inches to about 0.010 inches. In the case of the 1ml size syringe assembly, the diametric interference fit will preferably be about 0.005 inches or about 0.0025 inches radially. When the interference fit is in this range, the stress concentration occurring in the area of reduced thickness 60, when the plunger is preferably formed from polypropylene, will be below the yield point of polypropylene which is about 3,000 pounds per square inch. The sealing compression force developed for the 1ml size syringe assembly when the interference is in the preferred range of about 0.001 inches to about 0.010 inches will be between about 4 to about 6 pounds per square inch. For the 1ml syringe assembly, the circumferentially continuous portion 64 which forms the fluid tight seal 66 with the barrel wall will have an contact area about 0.0025 square inches to about 0.006 square inches. For the 1ml syringe assembly with the preferred interference and contact areas, the force required to slidably move the plunger for drawing and expelling fluid will be similar to the forces required to move an elastomeric plunger in a conventional single use 1ml syringe. One skilled in the art of injection molding and single use syringe assemblies will recognize that for other resins and other size syringe assemblies, that the interference relationships may be different.

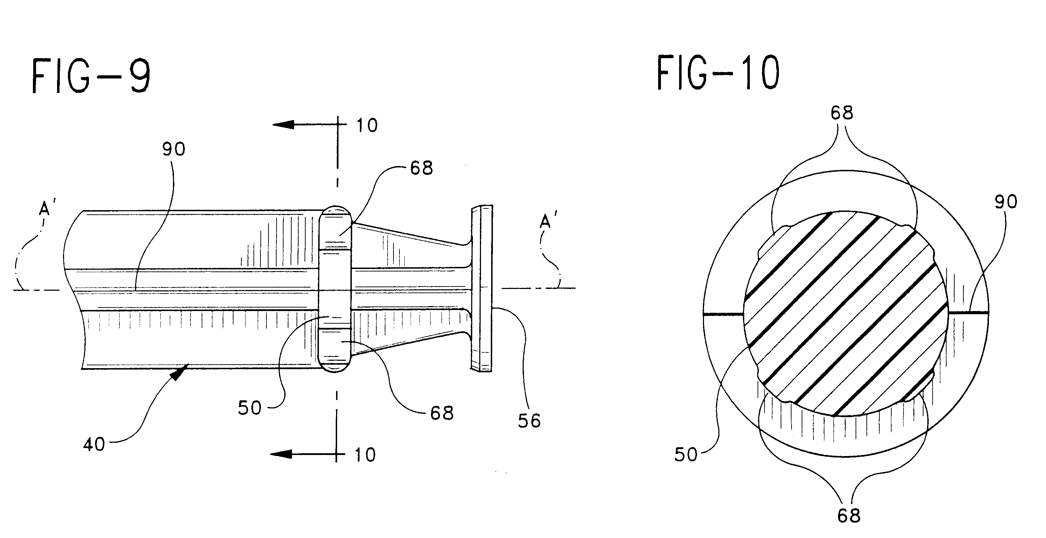

A similar observation may be made about the relationship between guide portion 50 and barrel wall 22. Guide portion 50 has a plurality of projections 68 which serve to keep the plunger axis substantially coaxial with the barrel axis. The projections also have an interference relationship with the barrel wall, preferably between about 0.001 inches to about 0.010 inches. In the case of the 1ml size syringe as shown in Fig. 10, there are preferably four projections transverse to the plunger axis symmetrically about the parting line from the mold with an interference preferably about 0.003 inches between the projections and the barrel wall.

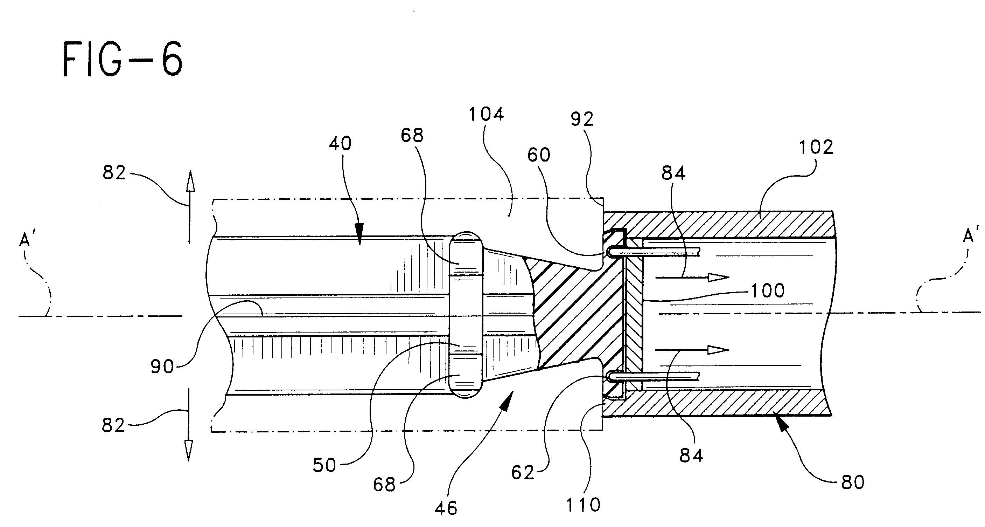

As shown in Figs. 6, 7, 8 and 9 in the design of the molding tool for the plunger of the syringe assembly of the present invention, it was discovered that there were several necessary considerations about the mold opening. The necessity to flex the area of the reduced thickness to form a living hinge, was described above. Additionally, it was discovered that the parting lines, which occur on a molded part as a result of the mold opening locations, could not be on any areas of the plunger of the present invention where the tolerance of the dimension is critical for forming an interference with the barrel. There are two critical locations for interference between the plunger and the barrel wall in the syringe assembly of the present invention. These critical areas are the circumferentially continuous portion 64 which contacts barrel wall 22 to form fluid tight seal 66 and the contact between projections 68 and barrel wall 22 to maintain plunger axis A' substantially coaxial with barrel axis A.

A molding tool 80 of the present invention has a primary mode 82 and a secondary mode 84 of opening. Molding tool 80 is useful for forming plunger 40 of the present invention from a thermoplastic resin using an injection molding process . A method for forming plunger 40 includes providing injection molding tool 80 which has a cavity therein for forming the plunger. The injection molding tool has a primary opening mode 82 which forms a primary parting line 90 along longitudinal axis A' of plunger 40 and secondary mode 84 of opening forms a secondary parting line 92 on sealing portion 48 of piston 46 located proximally to circumferentially continuous portion 64 of outside section 58 which forms fluid tight seal 66 with barrel wall 22. The injection molding tool is mounted in a suitable injection molding press and provided with a suitable thermoplastic resin. The press is operated in a normal fashion to melt the resin and fill the molding tool with resin so that the cavity for the plunger is filled. After the molding tool is filled with resin, sufficient quiescent time is allowed for the resin to cool below its melting point before the molding tool is opened to remove the formed plunger.

In an opening sequence for molding tool 80, secondary mode 84 occurs first. The secondary opening sequence begins by withdrawal of an insert 100 into a face section 102 of molding tool 80. Insert 100 forms area of reduced thickness 60 in the form of groove 62 and a portion 53 of distal surface 54. Secondary opening 84 then continues by distally axially withdrawing face section 102 away from primary section 104 of mold tool 80. When face section 102 of the molding tool moves distally axially, a raised section 110 of face section 102 causes a distal flexion of outside section 58 of the sealing portion with respect to central section 56 at area of reduced thickness 60 thereby orienting the polymer and forming a living hinge in the area of reduced thickness. Continuing the mold opening sequence, primary opening mode 82 then follows, providing access to the finished plunger 40. Preferably, projections 68 are located symmetrically away from the molding tool primary parting line 90 formed by primary opening mode 82 so that close tolerances may be held for the interference fit between projections 68 and barrel wall 22.

As shown in Fig. 10, in the preferred plunger rod 40 of the present invention for the 1ml syringe assembly, preferably there are four projections 68 with the plunger and the barrel being formed from polypropylene. Other satisfactory thermoplastic resins besides polypropylene for forming the plunger rod may include polyethylene, copolymers of polyethylene and polypropylene, polyvinylchloride and polystyrene. For other sizes of syringe assemblies and when other thermoplastic resins are used to form the plunger rod and barrel, the number of projections and the interference relationships between the projections and the barrel may change.

Additionally, a plunger of the present invention may have sealing portion 48 of the piston colored differently from guide portion 50 and the elongate shaft portion. The different color may be added after the molding process as a secondary coating applied to the sealing portion, a colored insert may be placed in the molding tool or the plunger may be molded using a two color process wherein the resin used to form the sealing portion is colored differently than the resin used to form the balance of the plunger. Preferably circumferentially continuous portion 64 of the sealing portion which forms fluid tight seal 66 provides a contrast between the fluid contained in chamber 24 of the syringe assembly to enable the dosage to be accurately measured using measuring indicia 32 on the syringe barrel.

Functionally, it is important that the axial movement of the plunger in the barrel of a syringe during filling and dispensing liquids be free and smooth. In most currently available single use syringe assemblies with resilient plunger stoppers and thermoplastic barrels, it is customary to apply a small amount of polydimethylsiloxane (silicone) to the inside of the barrel to facilitate movement of the plunger against the barrel wall. Additionally, slip agents such as fatty acid amides are often added to the resin used to mold the barrels. In the syringe assembly of the present invention, movement of piston 46 against barrel wall 22 may be facilitated by applying a small amount of silicone lubricant to barrel wall 22. In the present invention, when a silicone lubricant is used about 0.1 mg of polydimethysiloxane having a viscosity of between about 500 centistokes and about 12,500 centistokes may be used, with 1,000 centistokes being preferred. When a slip agent is chosen to facilitate movement of plunger in the barrel, the addition of fatty amide slip agents such as stearyl erucamide, erucyl erucamide, oleyl palmitamide, stearyl stearamide, erucyl stearamide and the like are usable. Suitable examples of these compounds are available from Humko, Memphis, TN, under the trade name Kemamide. Of these compounds, erucyl erucamide was a preferred additive in the 1ml syringe assembly, but addition of silicone lubricant is preferred over the use of slip agents.

Single use syringe assemblies are packaged in microorganism resistant packages then exposed to an environment which renders any microorganisms present in the package non-viable. This process is commonly referred to as sterilization. Sterilization processes commonly used are exposure to ethylene oxide or exposure to ionizing radiation. The materials used to form single use syringe assemblies must not be adversely effected by the sterilization process. For preferred syringe assemblies of the present invention, where the plunger and the barrel are formed from polypropylene, preferred polypropylene resins would include addition of an agent for radiation stabilization of the resin. Radiation stabilization agents commonly are hindered amines and other compounds having the ability to rapidly react with and quench free radicals generated by cleavage of polymer chains induced by ionizing radiation. Such radiation stabilized polypropylene resins are available from Himont Inc. Wilmington, DE, Exxon, Houston, TX and others. Preferred radiation stabilized polypropylene resins are Himont Pro-Fax resins PF-531, PF-511 and PF-091B having melt flows (ASTM method 1238) between about 20 and about 30 dg/min.

The syringe assembly of the present invention with the integrally formed plunger of the present invention is simpler to manufacture than currently available syringe assemblies. By eliminating the need for an elastomeric piston, the assembly has fewer parts, requires fewer materials to be inventoried, no elastomeric molding equipment, less assembly equipment and fewer assembly steps. The syringe assembly of the present invention should be considered an advancement in the art of single use syringes.

## Claims

1. A syringe assembly comprising:
a barrel including a cylindrical body portion having a longitudinal axis, said body portion forming an inside wall spaced from said axis defining a chamber having an inside diameter for retaining fluid, said body portion including an open proximal end and a distal end having a passageway thererethrough in fluid communication with said chamber; and
a plunger including an elongate shaft portion and having a longitudinal axis, a proximal end and a distal end comprising a piston, said piston being slidably positioned in said barrel, said piston including a distal sealing portion and a guide portion, said sealing portion having a thickness and being located distally to said guide portion, said sealing portion having a distal surface and further including a central section and an outside section, said sealing portion having an outside diameter (O.D.) greater than the inside diameter (I.D.)of said chamber before said piston is placed within said barrel, said sealing portion having an area of reduced thickness in the form of a groove in said distal surface, said area of reduced thickness flexibly joining said central section and said outside section so that when said piston is placed in said barrel at least a circumferentially continuous portion of said side section contacts said barrel wall and flexibly distally deflects said outside section relative to said central section at said area of reduced thickness allowing said piston to be fit within said barrel and thereby forming a fluid tight seal with said wall of said barrel facilitating drawing fluid into and expelling fluid from said chamber with a movement of said piston, said guide portion including a plurality of projections for contacting said inside wall of said chamber for maintaining said plunger axis substantially coaxial to said barrel axis, said proximal end of said plunger extending outwardly from said proximal end of said barrel to facilitate moving said piston with respect to said barrel.

2. The syringe assembly of claim 1 wherein said outside section O.D. is greater than said barrel I.D. and comprises an interference fit, said interference being between about 0.001 inches to about 0.010 inches.

3. The syringe of claim 1 wherein said plunger comprises a single article of manufacture integrally formed from a polypropylene resin.

4. The syringe of claim 3 wherein said polypropylene resin includes an additive selected from the group consisting of a slip agent, a radiation stabilizer and a combination of a slip agent and a radiation stabilizer.

5. The syringe of claim 1 wherein said elongate shaft of said plunger comprises a cruciform cross section substantially symmetrical about said longitudinal axis of said plunger.

6. The syringe of claim 1 wherein said guide portion of said of said piston comprises four projections substantially symmetrically arranged about said longitudinal axis of said plunger.

7. The syringe of claim 1 wherein said barrel is formed from a thermoplastic resin selected from the group consisting of polypropylene, polyethylene, polycarbonate and polystyrene.

8. The syringe of claim 7 wherein said barrel is rigid and substantially transparent and said thermoplastic resin comprises polypropylene including an additive selected from the group consisting of a slip agent, a radiation stabilizer and a combination of a slip agent and a radiation stabilizer.

9. A syringe assembly comprising:
a rigid substantially transparent barrel including a cylindrical body portion having a longitudinal axis. said body portion having a sidewall forming an inside wall spaced from said axis defining a chamber having an inside diameter for retaining fluid, said body portion including an open proximal end and a distal end having a passageway thererethrough in fluid communication with said chamber;
a hub portion including a fixedly attached hypodermic needle at said distal end of said barrel in fluid communication with said passageway;
volume measuring indicia on said sidewall; and
a plunger including an elongate shaft portion and having a longitudinal axis, a proximal end and a distal end comprising a piston, said piston being slidably positioned in said barrel, said piston including a distal sealing portion and a guide portion, said sealing portion having a thickness and being located distally to said guide portion substantially transverse to said longitudinal axis of said plunger, said sealing portion having a distal surface and further including a central section and an outside section, said sealing portion having an outside diameter greater than the inside diameter of said chamber before said piston is placed in said barrel. said sealing portion having an area of reduced thickness in the form of a groove in said distal surface, said area of reduced thickness flexibly joining said central section and said outside section so that when said piston is placed in said barrel at least a circumferentially continuous portion of said outside section contacts said inside wall and flexibly distally deflects said outside section relative to said central section at said area of reduced thickness allowing said piston to fit within said barrel and thereby forming a fluid tight seal with said inside wall of said barrel facilitating drawing fluid into and expelling fluid from said chamber with a movement of said piston, said guide portion including a plurality of projections for contacting said inside wall of said barrel for maintaining said plunger axis substantially coaxial to said barrel axis, said proximal end of said plunger extending outwardly from said proximal end of said barrel to facilitate moving said piston with respect to said barrel.

10. The syringe assembly of claim 9 wherein said plunger is formed from a thermoplastic resin by an injection molding process in a mold tool having a primary mode of opening forming a primary parting line along said longitudinal axis corresponding to the elongate shaft and a secondary mode of opening forming a secondary parting line on said sealing portion of said piston transverse said longitudinal axis and located proximally to said circumferentially continuous portion of said outside section which contacts said inside wall of said barrel. said area of reduced thickness comprising a living hinge being formed by a distal flexion of said outside section with respect to said central section at said area of reduced thickness by said secondary mode of said mold opening and said guide portion having a plurality of projections for contacting said inside wall of said barrel, said projections being substantially symmetrically arranged about said primary parting line.
